# EUROPEAN PATENT APPLICATION

(11) **EP 4 198 515 A1**
(43) Date of publication of application: **21.06.2023**
(21) Application number: 21306765.5
(22) Date of filing: 14.12.2021
(51) Int. Cl.: G01N 33/80

(54) **METHODS AND KITS FOR IDENTIFYING AND QUANTIFYING STORAGE-INDUCED MICROERYTHROCYTES**

(71) Applicant: INSERM (Institut National de la Santé et de la Recherche Médicale), 75013 Paris (FR); Université de Paris, 75006 Paris (FR)
(72) Inventor: The designation of the inventor has not yet been filed
(74) Representative: Inserm Transfert

(57) **Abstract**

Refrigerated storage of red cell concentrates (RCC) for transfusion is associated with the accumulation of various alterations to the red blood cells (RBCs). Among these, a subpopulation of small RBCs defined as storage-induced microerythrocytes (SMEs) accumulates during storage. The SMEs subpopulation correlates with transfusion recovery. Quantification of this morphologically-altered RBC subpopulation using flow cytometry would be a valuable tool to evaluate RCC quality. In the present invention, RBC obtained at the beginning or at the end of storage from RCC stored in SAGM in blood bank conditions were treated with a carboxyfluorescein diacetate succinimidyl ester (CFDA-SE) staining protocol and were finally analysed by flow cytometry to assess the intensity of CFSE staining. The inventors observed the accumulation of a CFDA-SE^{high} subpopulation by flow cytometry that accounted for 0.8% and 36.3% at day 3 and 42 of storage, respectively. Images confirmed that the CFDA-SE^{high} subpopulation mostly contains SMEs. Thus SMEs can now be simply quantified using a common fluorescent dye and a standard flow cytometer.

## Description

### FIELD OF THE INVENTION:

The present invention is in the field of medicine, in particular haematology.

### BACKGROUND OF THE INVENTION:

During hypothermic pre-transfusion storage, part of the RBC metabolism shifts from glycolysis to the pentose phosphate pathway after 10-14 days of storage, leading to a progressive decrease of the intracellular ATP level (D'Alessandro et al, Transfusion, 2015; Bordbar A et al, Transfusion, 2016). The fragilized RBC is thus less capable of coping with the oxidative stress present in the bag further contributing to fragilize the RBC integrity (Reisz JA et al, Blood, 2016). These metabolic and oxidative stresses contribute to the progressive modifications of RBC properties (Yoshida T et al, Blood Transfus, 2019). Among those, RBC morphology is a well-documented and key RBC property altered during storage.

A number of techniques are used to explore RBC morphology. The pioneer work of Bessis and his collaborators using scanning electron microscopy defined seven RBC morphology classes (Bessis M, Nouvel Rev Fran Hematol, 1972) (Bessis M, livre Corpuscules, 1974). This technique is still used to assess RBC morphology during storage (Berezina, 2012; D'Alessandro, haematologica, 2012; Blasi, 2012; Antonelou MH, Journal of Proteomics, 2012; Roussel, Morel, 2021; Zehnder L et al, Vox Sang, 2008).

Digital holographic microscopy was recently used to explore RBC morphology along storage (Bardyn, Blood Transfus, 2017). Light microscopy can also be used to observe fixed (Högman, Vox Sang, 1985; Tchir, Transfusion, 2013; Reinhart, Transfusion, 2015) or unfixed cells in a physiological medium (Lu and shevkoplyas, Transfusion, 2020; Roussel, Transfusion, 2017). RBC morphology is indeed sensitive to RBC intrinsic properties and to the suspension medium (Bessis M, Nouvel Rev Fran Hematol, 1972). Imaging flow cytometry rapidly acquires tens of thousands of images of unfixed cells in a physiological medium and identifies a well-demarcated subpopulation of morphologically altered and smaller RBCs, comprising type III echinocytes, spheroechinocytes, and spherocytes (Roussel, 2017). These small RBCs, defined as storage-induced micro-erythrocytes (SMEs), accumulate during storage, reaching a mean proportion of 25% of the entire RBC population at day 42 of storage and negatively correlate with post-transfusion recovery in healthy volunteers (Roussel, 2021). Furthermore, the Imaging Flow Cytometry capacity to discriminate fluorescently-stained cells allowed to observe their clearance from circulation after ex vivo perfusion in a human spleen and in vivo transfusion in mouse model (Roussel, 2021). Quantification of morphologically-altered RBCs is thus a relevant storage quality marker that identifies the RBC subpopulation targeted for rapid post-transfusion clearance. Quantification of morphologically-altered RBCs using flow cytometry would be simpler. Also, obtaining purified preparations of these cells could allow to investigate the intrinsic properties of RBC that lead to their clearance.

### SUMMARY OF THE INVENTION:

The present invention is defined by the claims. In particular, the present invention relates to methods and kits for identifying and quantifying storage-induced microerythrocytes (SME).

### DETAILED DESCRIPTION OF THE INVENTION:

Refrigerated storage of red cell concentrates (RCC) for transfusion is associated with the accumulation of various alterations to the red blood cells (RBCs). Among these, a subpopulation of small RBCs, comprising type III echinocytes, sphero-echinocytes, and spherocytes and defined as storage-induced micro-erythrocytes (SMEs) accumulate to reach approximately 25% of RBC on day 42 of storage. The SMEs subpopulation is variable between donors, is cleared rapidly after transfusion, correlates with transfusion recovery and can be rapidly and objectively quantified using imaging flow cytometry. Selection and quantification of this morphologically-altered RBC subpopulation using flow cytometry would be a valuable tool to evaluate RCC quality but a selective stain is lacking. The inventors obtained RBC at the beginning or at the end of storage from RCC stored in SAGM in blood bank conditions. The RBC were then treated with a carboxyfluorescein diacetate succinimidyl ester (CFDA-SE) staining protocol and were finally analysed by flow cytometry to assess the intensity of CFDA-SE staining. In parallel, SMEs were quantified by imaging flow cytometry. The inventors observed the accumulation of a CFDA-SE^{high} subpopulation by flow cytometry that accounted for 0.8% and 36.3% at day 3 and 42 of storage, respectively. Imaging flow cytometry showed a mean projected area of 45.6 µm² and 78.2 µm² for CFDA-SE^{high} and CFDA-SE^{low} subpopulations, respectively. Images confirmed that the CFDA-SE^{high} subpopulation mostly contains SMEs while the CFDA-SE^{low} subpopulation mostly contains type I and II echinocytes and discocytes. Similar numbers of SMEs were quantified by imaging flow cytometry (based on projected surface area of RBC) and by conventional flow cytometry (based on CFDA-SE intensity). CFDA-SE^{high} and CFDA-SE^{low} subpopulations could be sorted by flow cytometry and contained >95% and <5% SMEs, respectively, by imaging flow cytometry and scanning electron microscopy. SMEs can now be simply quantified using a common fluorescent dye and a standard flow cytometer. This simple staining protocol enables the specific sorting of SMEs, a useful tool to further characterize this RBC subpopulation targeted for clearance after transfusion.

The present invention relates to a method for detecting the presence of storage-induced microerythrocytes (SMEs) in a red blood cell containing composition comprising the steps of i) staining the composition with an amount of a cell permeable dye, ii) incubating the stained composition for a sufficient period of time and under conditions suitable for allowing a bimodality of staining to appear in the red blood cells, and iii) distinguishing the cells by their intensity of staining wherein the subpopulation of SMEs are characterized by a high intensity of staining.

As used herein, the term **"erythrocytes"** or **"red blood cells"** or **"RBCs"** has its general meaning in the art and refers to highly-specialized cells responsible for delivery of oxygen to, and removal of carbon dioxide from, metabolically-active cells via the capillary network. They are shaped as biconcave discs and average about 8-10 microns in diameter.

A used herein, the term **"storage-induced microerythrocytes"** or **"SMEs"** has its general meaning in the art and refers to a subpopulation of small RBCs, comprising type III echinocytes, spheroechinocytes, and spherocytes. During storage, RBCs indeed show various stages of morphological deterioration, including echinocyte 1 (E1), echinocyte 2 (E2), echinocyte 3 (E3), sphero-echinocyte (SE), spherocyte (S) and stomatocyte (ST).

As used herein, the term **"red blood cell containing composition"** means whole blood, red blood cell concentrates and any other composition that contains red blood cells. Typically, the red blood cell containing composition is an individual stored red blood cell (RBC) unit. Other than red blood cells, the composition can also contain a biologically compatible solution, such as SAGM, PAGGSM, ARC-8, Nutricell (AS-3), ADSOL (AS-1), Optisol (AS-5) or RAS-2 (Erythrosol), and one or more cellular blood components, one or more blood proteins, or a mixture of one or more cellular blood components and/or one or more blood proteins. Such compositions may also contain a liquid blood component, such as plasma. Typically, the red blood cell containing composition was stored for a period of 1 to 42 days at a temperature of 2 to 6°C, preferably at 4°C.

As used herein, the term **"cell permeable dye"** has its general meaning in the art and refers to a dye that freely diffuses through the membranes of live cells. In some embodiments, the dye is a fluorescent dye. As used herein, the term **"fluorescent dye"** means a dye which absorbs light at a first wavelength and emits at second wavelength which is longer than the first wavelength. Fluorescent dyes typically include fluorescent compounds having a chemically reactive group that facilitates attachment of dye to a conjugate molecule. Numerous fluorescence dyes useful for selective labeling of viable cells are described in the art. In particular, the dye is an **"amine tracer"** wherein upon reaction with amine-containing residues of intracellular proteins, the resulting dye-protein adducts are well retained in cells.

In some embodiments, the dye is a succinimidyl ester-based dye that binds to the amine groups of cellular proteins. For instance, succinimidyl esters can be prepared simply by reacting fluorescent dyes bearing carboxyl groups with N-hydroxysulfosuccinimides. In some embodiments, the dye is selected from the group consisting of carboxyfluorescein diacetate succinimidyl ester (CFDA-SE), carboxyfluorescein succinimidyl ester (CFSE), carboxyeosin diacetate succinimidyl ester or any derivatives thereof. For instance, the dye can also be selected from CellTrace^{™} dyes (e.g. CellTrace Violet^{™}, CellTrace^{™} Far Red). In some embodiments, the dye is carboxyfluorescein diacetate succinimidyl ester (CFDA-SE, or, alternatively, CFSE) that is excitable with 488 nm laser light to give a bright green fluorescence with a maximum emission at 517 nm. The term **"CFDA-SE"** has its general meaning in the art and refers to 2,5-Dioxopyrrolidin-1-yl 3' ,6' -bis(acetyloxy)-3-oxo-3H-spiro[[2]benzofuran-1,9' -xanthene]-6-carboxylate.

In general, the dye is provided to the cells at a concentration ranging from about 0.01µM to about 1µM, preferably 0,05µM. Typically, the incubation step is carried out at 37°C and for at least 10, 15, 20, 25 or 30 minutes preferably 20 minutes. Typically, the cells once stained are centrifugated and washed and then incubated in an appropriate culture medium (e.g. RPMIc) for at least 4h, typically overnight. Following incubation, the cells are centrifuged, resuspended in a fresh culture medium and stored until analysis.

After being contacted with the fluorescent dye, the composition is excited by a light source capable of producing light at or near the wavelength of maximum absorption of the fluorescent complex, such a laser, an arc lamp, an ultraviolet or visible wavelength emission lamp. Any apparatus or device that can measure the total fluorescence of a sample can be used in this invention including flow cytometers. Typically, the fluorescence emitted by the fluorescent dye is measured by means of a flow cytometer, such as a Fluorescence Activated Cell Sorter (FACS) machine. As used herein, the term **"fluorescence activated cell sorting"** or **"FACS"** refers to a method by which the individual cells of a sample are analysed and sorted according to their optical properties (e.g., light absorbance, light scattering and fluorescence properties, etc.) as they pass in a narrow stream in single file through a laser beam. Fluorescence-activated cell sorting is a specialized type of flow cytometry. It provides a method for sorting a heterogeneous mixture of biological cells into two or more containers, one cell at a time, based upon the specific light scattering and fluorescent characteristics of each cell. It is a useful scientific instrument as it provides fast, objective and quantitative recording of fluorescent signals from individual cells as well as physical separation of cells of particular interest. In a typical FACS system, the cell suspension is entrained in the centre of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell being in a droplet. Just before the stream breaks into droplets the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement and the opposite charge is trapped on the droplet as it breaks from the stream. The charged droplets then fall through an electrostatic deflection system that diverts droplets into containers based upon their charge. In some systems the charge is applied directly to the stream and the droplet breaking off retains charge of the same sign as the stream. The stream is then returned to neutral after the droplet breaks off. The fluorescent labels for FACS technique depend on the lamp or laser used to excite the fluorescent dye and on the detectors available.

In some embodiments, the fluorescent dye intensity is expressed as an absolute value (e.g. fluorescence intensity) or as a rate (e.g. fluorescence intensity by a section of time). In some embodiments, the fluorescence intensity is expressed as MFI. The term **"MFI",** as used herein, refers to the mean or median fluorescence intensity of a population of fluorescent cells.

As used herein, the expression **"high intensity of staining"** indicates that intensity of the staining is high by comparison with the staining intensity in whole population of cells. The term **"high"** thus indicates that the intensity is higher than the mean or median of intensity measured in the whole population of cells. Typically, when the CFDA-SE dye is used the population of SMEs is defined as the CFDA-SE^{high} fraction.

In some embodiments, the method of the present invention comprises the step of assessing the size of the cells. According to the present invention the SMEs are indeed characterized by a small size and a high intensity of staining. The term **"small size"** typically indicates that the size of the cells is lower than the mean or median of size measured in the whole population of cells.

In some embodiments, FACS brings the advantage that by using a gating strategy it is possible in the same sample to determine the fluorescent dye intensity and the size of the cells. Typically, a strategy of gating is described in the EXAMPLE for identifying the population of SMEs.

In some embodiments, the method of the present invention comprises the step of quantifying the amount of SMEs present in the red blood cell containing composition.

The method of the present invention is particularly suitable for quantifying the quality degradation of red blood cell containing compositions, and processing this data to facilitate better-informed medical decisions regarding units' respective allocation, patient suitability, and use. Typically deploying the method across a hospital's inventory or even throughout the supply chain will improve distribution, planning, and inventory control decisions. In particular, the method of the present invention is of particular interest in the context of chronically transfused patients for which the poor transfusion recovery could have a major impact. For example, transfusion-related iron overload is a major cause of morbidity and mortality in these patients, and the need to provide more transfusions because some are of lower quality or recovery could have significant adverse consequences. Thus, the method of the present invention is of a particular interest for patients suffering from haematological conditions such as β-hemoglobinopathies such as sickle cell disease or thalassemia (e.g. β-thalassemia).

Accordingly, a further object of the present invention relates to a method of assessing the storage quality of a red blood cell containing solution comprising quantifying the amount of SMEs present in the composition by the quantifying method herein disclosed wherein the amount of SMEs correlates with the quality storage.

As used herein, **"storage quality"** is defined as the extent of post-transfusion recovery of the stored RBCs; higher recovery is defined as higher quality. Examples of post-transfusion recovery include greater than zero and almost 100% recovery, i.e., recovery of 10%, 20%, 30%, 40%, 50%, 60%, 70%, 80%, 90%, 100%, and all percentages in between. In some embodiments, an acceptable storage quality is an average of 75% post-transfusion recovery at 24 hours, as under FDA guidelines.

Typically, the higher is the amount of SMEs present in the composition, the worse is the storage quality of the red blood cell composition. In particular, red blood cell containing composition is considered as being of good quality when at least about 70%, preferably at least about 75%, still preferably at least about 80%, 85%, advantageously 90%, 91 %, 92%, 93%, 94%, and ideally 95%, 96%, 97%, 98%, 99% or 100% of the RBCs are not morphologically altered in SMEs.

In particular, the method of the present invention is also particularly suitable for predicting the transfusion recovery of a patient. It is established that when the subpopulation of SMEs was abundant in RBC concentrates, transfusion recovery is diminished. Typically, the patient is a chronically transfused patient and more particularly a patient suffering from a β-hemoglobinopathy.

Accordingly, a further object of the present invention relates to a method of predicting the transfusion recovery of a patient transfused with a red blood cell containing composition comprising quantifying the amount of SMEs present in the composition by the quantifying method herein disclosed wherein the amount of SMEs correlates with the recovery yield. More particularly, the method comprises the steps of i) quantifying the amount of SMEs present in the red blood cell containing solution, ii) comparing the amount quantified at step i) with a predetermined reference value, iii) and concluding that the patient will a have a good recovery when the amount quantified at step i) is lower than the predetermined reference value or concluding that the patient will have a poor recovery when the amount quantified at step i) is higher than the predetermined reference value.

Typically, the predetermined reference value is a threshold value or a cut-off value. A **"threshold value"** or **"cut-off value"** can be determined experimentally, empirically, or theoretically. A threshold value can also be arbitrarily selected based upon the existing experimental and/or clinical conditions, as would be recognized by a person of ordinary skilled in the art. For example, retrospective quantification of SMEs in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the amount of SMEs in a group of reference, one can use algorithmic analysis for the statistic treatment of the measured amounts of SMEs in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc. In some embodiments, a cut-off value consists of a range of quantification values, e.g. centered on the quantification value for which the highest statistical significance value is found. For example, on a hypothetical scale of 1 to 10, if the ideal cut-off value (the value with the highest statistical significance) is 5, a suitable (exemplary) range may be from 4-6. For example, a patient may be assessed by comparing values obtained by measuring the amount of SMEs, where values greater than 5 reveal that the patient is at risk of having a poor recovery and values less than 5 reveal that the patient will have a good recovery. In some embodiments, a patient may be assessed by comparing values obtained by measuring the amount of SMEs and comparing the values on a scale, where values above the range of 4-6 indicate that the patient is at risk of having a poor recovery and values below the range of 4-6 indicate that the patient will have a good recovery, with values falling within the range of 4-6 indicate that further explorations are needed to conclude whether the patient is at risk of having a poor recovery.

The method of the present invention is also particularly suitable for preparing a red blood cell containing composition suitable for transfusion comprising i) detecting the presence of SMEs in red blood cell containing composition and ii) separating the SMEs from blood cell containing composition. Any sorting method as described above (e.g. FACS) can be used for said preparation.

Also encompassed within the scope of the invention are test kits for performing the methods of the present invention. Complete test kits contain solutions and devices quantifying the amount of SMEs present in a red blood cell containing composition. For example, the test kit contains a 96, 384, or 1536 well plate for high throughput sample purification, and/or solutions for staining the population of red blood cells with the cell permeable dye. Generally, the test kits of the invention contain one or more of the following: (1) one or more containers (2) one or more solution containing the cell permeable dye for staining the red blood cells; (3) instructions for practicing the methods described herein; (4) one or more assay component; and (5) packaging materials. In some embodiments, the kits include in addition to one or more of the, buffers, reagents, chemical agents, functionalization reagents, enzymes, detection agents, control materials, or the like, among others. Other items which may be provided as part of the test kit include solid surface syringes, pipettes, cuvettes, and containers.

The invention will be further illustrated by the following figures and examples. However, these examples and figures should not be interpreted in any way as limiting the scope of the present invention.

### FIGURES:

**Figure 1****: Bimodality in Carboxyfluorescein diacetate succinimidyl ester (CFDA-SE) reactivity in RBC stored in blood bank conditions.**
   Representative frequency plots of fluorescence intensity for RBC stored for 42 days in SAGM, either immediately after CFDA-SE staining (grey histogram) and during the 24 hours of incubation at 37°C (black line).
**Figure 2****: CFDA-SE^{high} cells correspond to morphologically-altered RBCs that accumulate during pretransfusion storage.**
   (A) Representative CFDA-SE intensity frequency plots of unstained (grey histogram) and CFDA-SE stained (black line) long-stored RBCs, from imaging flow cytometry analysis, allowing to select CFDA-SE^{low} (dotted line) and CFDA-SE^{high} (dashdotted line) subpopulations by the nadir point of the bimodality of frequency plots. (B) Comparison of the mean CFDA-SE fluorescence intensities of each subpopulation obtained from 8 RCCs stored 42 days and determined by imaging flow cytometry. (C) Representative projected surface area frequency plots of the CFDA-SE^{low} and CFDA-SE^{high} subpopulations previously selected in A. (D) Comparison of the mean projected surface area from the CFDA-SE subpopulations previously selected in B from the 8 long-stored RCCs. Representative ImageStream brightfield and fluorescence images from CFDA-SE^{high} (E) showing mostly echinocytes III, spheroechinocytes and spherocytes, and CFDA-SE^{low} subpopulations (F) showing discocytes, echinocytes I and echinocytes II. Scale bars represent 7µm. Results are presented as mean ± SD in B and D and tests of Wilcoxon for non-parametric and paired data were applied to compare groups between each other (**: p = 0.0078).

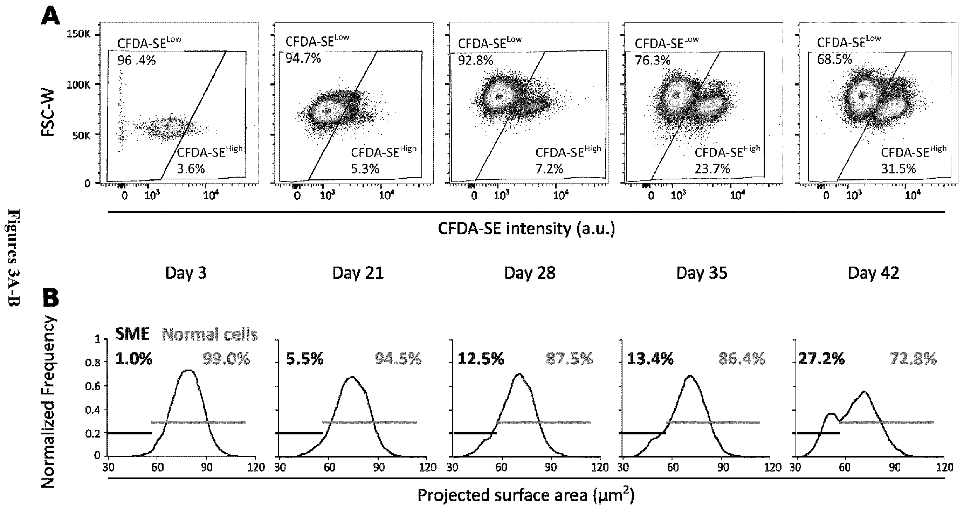

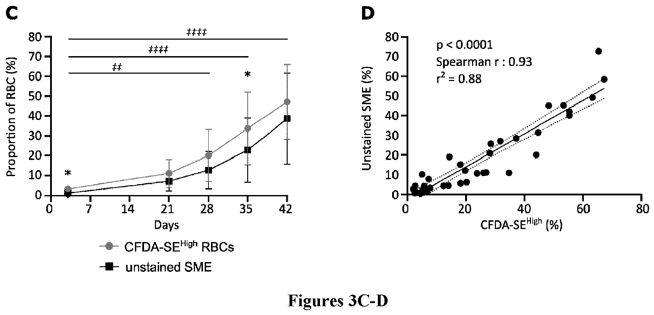

**Figure 3****: CFDA-SE^{high} staining allows quantification by flow cytometry of morphologically-altered RBCs along storage**
   (A) Representative density plots allowing quantification of CFDA-SE^{low} and CFDA-SE^{high} subpopulations along storage by flow cytometry. (B) Representative projected surface area frequency plots for unstained RBCs allowing quantification of morphologically-normal (grey line) and morphologically-altered (SME, black line) RBCs along storage by imaging flow cytometry. (C) Evolution of the proportion of the CFDA-SE^{high} (grey line) and unstained SMEs subpopulations (black line) during RCC storage (n=8). (D) Correlation between the two quantification techniques of morphologically-altered RBCs that accumulate along storage (Spearman r = 0.93, p < 0.0001; r² = 0.88). Results in C are represented as mean ± SD (vertical bars) and a two-way ANOVA, with the Geisser-Greenhouse correction followed by a Sidak's multiple comparison test, compared both techniques at each time point (*: p < 0.05) or the accumulation of CFDA-SE^{high} subpopulation along storage vs day 3 (*‡‡*: p = 0.0011 ; *‡‡‡‡*: p < 0.0001).

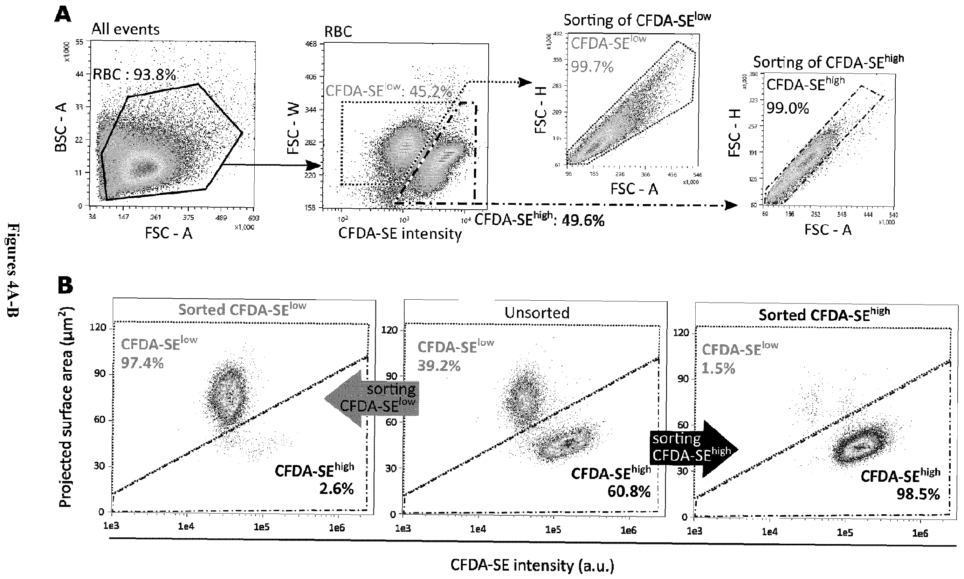

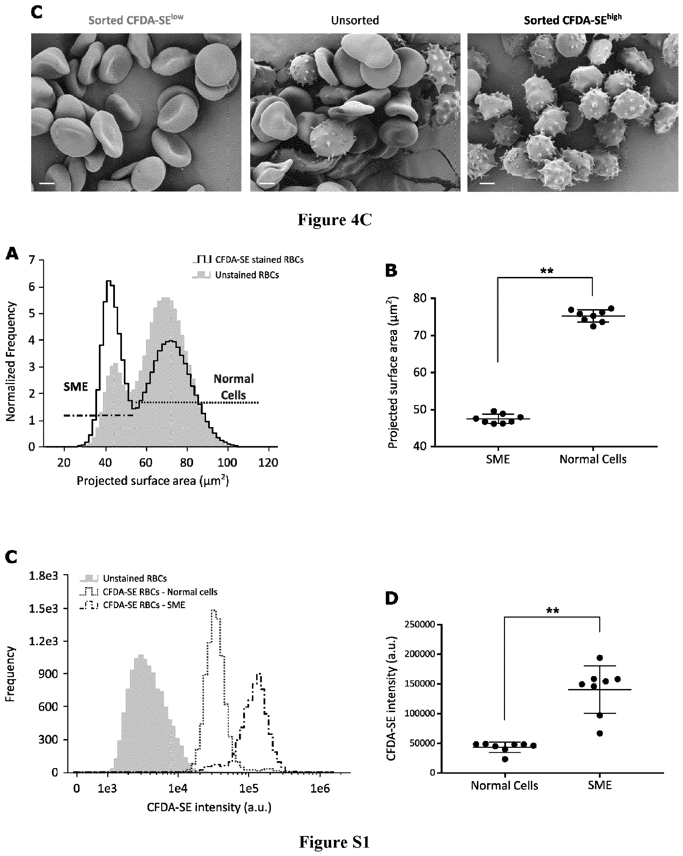

**Figure 4****: Enriched preparations of morphologically-normal and SMEs subpopulations of stored RBCs can be obtained by flow cytometry sorting.**
   (A) Flow cytometry sorting gating strategy, using fluorescence intensity and morphological parameters to select CFDA-SE^{low} (grey dotted line) and CFDA-SE^{high} (black dashdotted line) RBC subpopulations. Morphological criteria comprised the forward scatter (FSC) or back scatter (BSC) signals and their respective parameter of area (A), height (H) and width (W). (B) Representative imaging flow cytometry density plots of unsorted (middle panel), sorted CFDA-SE^{low} (left panel) and CFDA-SE^{high} (right panel) subpopulations illustrating each preparation purity. (C) Scanning electron microscopy images showing the morphology of RBC content in each unsorted or sorted fractions. Scale bars represent 2µm.
**Figure S1: Morphologically-altered RBCs that accumulate during pretransfusion storage show higher CFDA-SE staining intensity**
   (A) Representative projected surface area frequency plot, obtained by imaging flow cytometry, of unstained (grey histogram) and CFDA-SE stained (black line) long-stored RBCs segregating SME (dashdotted line) and normal cells (dotted line) subpopulations by the nadir point of the bimodal distribution. (B) Comparison of the mean projected surface area of SME and normal cells subpopulation determined as in A, from 8 RCCs stored 42 days. (C) Representative CFDA-SE intensity frequency plots of unstained (grey histogram) and the SME (dashdotted line) and normal cells (dotted line) subpopulations previously selected in A. (D) Comparison of the mean CFDA-SE fluorescence intensities of each SME and normal cells subpopulations determined as in C obtained from 8 RCCs stored 42 days. Results are presented as mean ± SD in B and D and tests of Wilcoxon for non-parametric and paired data were applied to compare groups between each other (**: p = 0.0078).
**Figure S2: Selection of the gating strategy to quantify morphologically-altered CFDA-SE^{high} and morphologically-normal CFDA-SE^{low} subpopulations of stored RBCs**
   (A) Representative imaging flow cytometry dot plot of projected surface area and fluorescence intensity of CFDA-stained RBCs after 42 days of storage. Selection using only fluorescence intensity (vertical dotted black bold line) shows that (B) CFDA-SE^{low} subpopulation contains some morphologically-altered RBCs and that (C) CFDA-SE^{high} subpopulation contains morphologically-normal RBCs. (D) Selection on morphological (projected surface area with imaging flow cytometry) and fluorescence (CFDA-SE intensity) criteria (oblique dotted black bold line) improves the gating specificity.

### EXAMPLE:

### Methods

### RBC concentrate collection and storage

Eight leukoreduced RBC concentrates provided by the Etablissement Français du Sang Haut de France-Normandie (French blood banking system) from healthy donors were stored in saline-adenine-glucose-mannitol (SAGM) solution at 2 to 6°C for 42 days. Samples were aseptically collected and analyzed at defined time-points (days 3, 21, 28, 35 and 42).

### CFDA-SE staining

RBC were washed once in phosphate buffer saline (PBS) and stained with CFDA-SE (5.5 millions RBC /mL, 0.05µM CFDA-SE in PBS) for 20 minutes at 37°C. RBCs were then centrifuged, washed once in RPMIc (RPMI 1640 supplemented with 10% FBS, 1% Antibiotic/Antimycotic solution) and incubated overnight in RPMIc at 22 millions RBC/mL. Following incubation, RBCs were centrifuged, resuspended in a fresh RPMIc solution and stored at 4°C until analysis.

### Flow cytometry analysis

CFDA-SE-stained RBCs were analyzed using FACScanto II (BD Biosciences) by recording 50,000 events. First, single cells were selected using morphological parameters (FSC-H vs FSC-A). CFDA-SE^{low} and CFDA-SE^{high} subpopulations were gated according to their size (FSC-W) and their CFDA-SE intensity (collected in FITC channel).

### Imaging flow cytometry analysis

Imaging flow cytometry was performed by ImageStream X Mark II (Amnis of EMD Millipore) to determine RBC dimensions and morphology as described (ROUSSEL C. et al, Transfusion 2017). RBC were suspended at 1% hematocrit (Hct) just before acquisition in a Krebs-albumin solution (Krebs-Henseleit buffer, Sigma-Aldrich) modified with 2 g of glucose, 2.1 g of sodium bicarbonate, 0.175 g of calcium chloride dehydrate, and 5 g of lipid-rich bovine serum albumin (Albu-MAX II, Thermo Fisher Scientific) for 1 L of sterile water (pH 7.4). Images (x60 magnification) were recorded (INSPIRE software, AMNIS) by the brightfield and FITC channels to be then processed by a dedicated computer software (IDEAS [version 6.2]; Amnis). Focused cells and single cells were respectively selected using the features gradient RMS_M01_Ch01 and Aspect ratio_M01_Ch01 versus Area_M01_Ch01. Front views were selected using the feature Circularity_Object (M01, Ch01, Tight) and projected surface area was determined using the feature Area Object (M01, Ch01, Tight). At least 6000 front views of focused single RBC/condition were analyzed. SME proportion and CFDA-SE intensity were determined independently for each donor, using the nadir of the bimodal frequency histograms as the gating boundary.

### Cell sorting

Sorting of CFDA-SE^{low} and CFDA-SE^{high} cells was performed using MA900 Cell Sorter (Sony) with a 100µm sorting-chip at the maximum speed of 10,000 events per second in semi-purity mode. Unsorted RBCs were selected using BSC-A vs FSC-A then CFDA-SE^{low} and CFDA-SE^{high} subpopulations were gated according to their size (FSC-W) and their CFDA-SE intensity (collected in FITC channel). Cell doublets were excluded by using FSC-H *vs* FSC-A and target cells were collected in tubes containing 1mL of RPMIc, then centrifuged and resuspended in RPMIc to be stored at 4°C until analysis.

### Scanning electron microscopy

Samples were fixed by incubation of minimum 24h in a mix of 4% paraformaldehyde and 1% glutaraldehyde diluted in 0.1 M of phosphate buffer (pH, 7.3) at 4°C, then washed in phosphate buffer, and postfixed by 1-hour incubation with 2% osmium tetroxide. Samples were then fully dehydrated in a graded series of ethanol solutions and dried by hexamethyldisilazane. Finally, samples were coated with 4 nm of carbon using a GATAN PECS 682 apparatus before observation under a Zeiss Ultra plus field emission-scanning electron microscope (ZEISS).

### Statistical analysis

Data were analyzed using GraphPad Prism version 9.2.0 for Windows (GraphPad Software, San Diego, California USA). To compare two groups of subpopulations (CFDA-SE^{low} vs CFDA-SE^{high} intensities or SME vs normal cells), Wilcoxon tests, for non-parametric and paired data, were applied. To compare means of CFDA-SE^{high} RBCs over time, two-way ANOVA with the Geisser-Greenhouse correction was performed with Sidak's multiple comparison test. Simple linear regression and a correlation test of Spearman were used to assess correlation between the two quantification techniques of CFDA-SE^{high} RBCs and SMEs. A P-value < 0.05 was considered statistically significant.

### Results:

### Bimodality in Carboxyfluorescein diacetate succinimidyl ester (CFDA-SE) reactivity in RBC stored in blood bank conditions

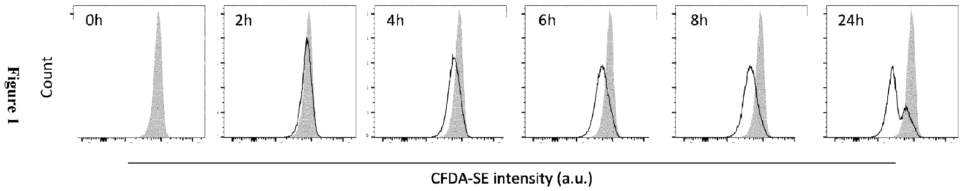

RBC, sampled from long-stored RBC concentrates, were stained using CFDA-SE and then incubated at 37°C in RPMIc medium. Immediately after CFDA-SE staining fluorescence intensity exhibited a unimodal distribution (0h, grey histogram, Figure 1). Incubation of the labeled RBC at 37°C lead to a progressive decline of fluorescence intensity in a subpopulation of RBC (black curves, Figure 1). Following an overnight incubation, the CFDA-SE intensity showed a clear bimodality separating two RBC subpopulations (Figure 1). The bimodality in CFSE-DA staining was not visible when fresh RBCs were observed or when RBCs were incubated at 4°C after staining (Figure S1). These results show that the CFDA-SE staining protocol with an overnight incubation identifies a discrete RBC subpopulation present in long-stored RBC.

### CFDA-SE^{high} cells correspond to morphologically-altered RBCs that accumulate during pretransfusion storage

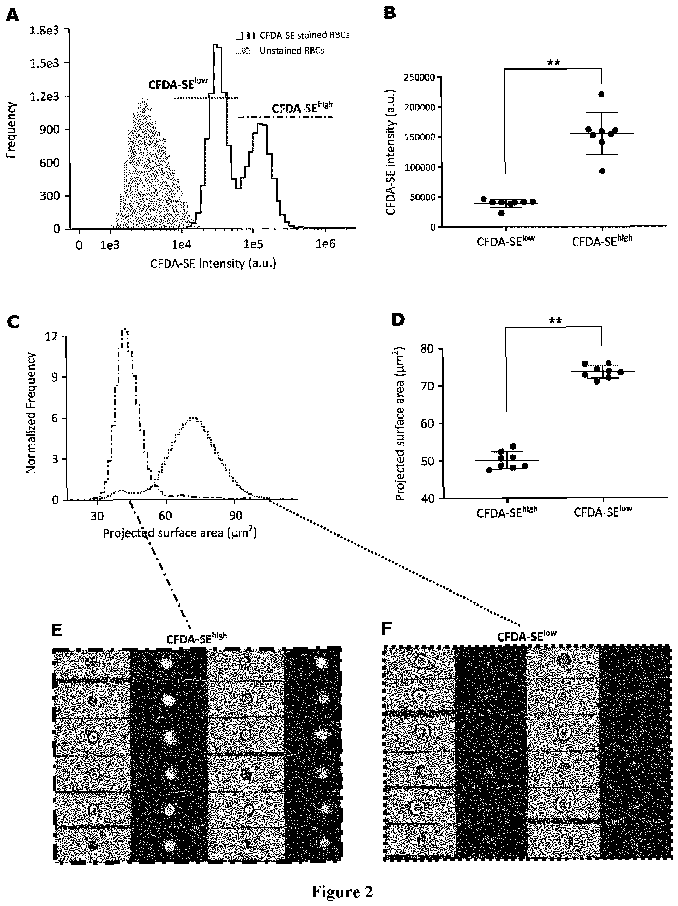

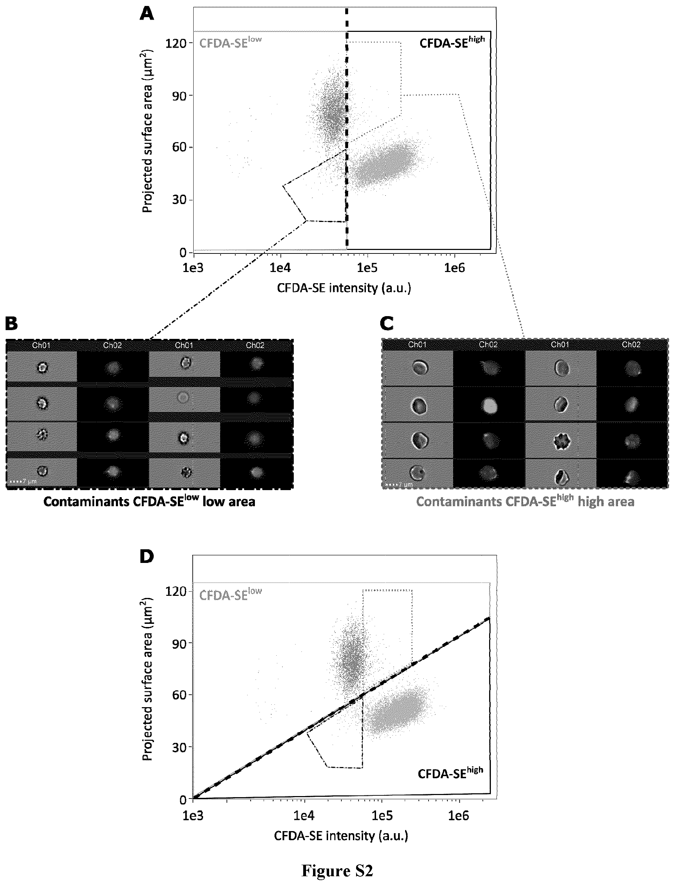

We next used imaging flow cytometry, that enables simultaneous analysis of fluorescence and morphological parameters, on 8 RBC concentrates stored for 42 days. A bimodal distribution of CFSE-DA staining was detected (black line, Figure 2A) and visible in 8/8 RBC concentrates. Segregating CFDA-SE^{low} RBC (dotted line, Figure 2A) from CFDA-SE^{high} RBC (dashdotted line, Figure 2A) was performed independently for each donor, using the nadir of the bimodal frequency histograms as the gating boundary. The mean fluorescence intensity of CFDA-SE^{high} RBCs (154729 a.u. ± 34931) was statistically higher than for the CFDA-SE^{low} subpopulation (39055 a.u. ± 6980; p = 0.0078; Figure 2B).

We next evaluated the projected surface area of each subpopulation to discriminate morphologically-altered RBC, the storage-induced micro-erythrocytes (SMEs), from morphologically-normal RBC along storage (Roussel, 2017; Roussel, 2021). Comparison of the distribution of projected surface area between the two subpopulations showed that CFDA-SE^{high} RBCs have a lower projected surface area than RBCs from the CFDA-SE^{low} subpopulation (Figure 2C). Mean projected surface area of CFDA-SE^{high} RBCs (50.1 µm²±2.2) was significantly lower than for CFDA-SE^{low} RBCs (73.7 µm² ±1.7; p = 0.0078; Figure 2D). Morphologic analysis of imaging flow cytometry brightfield images showed that the CFDA-SE^{high} subpopulation contained a majority of echinocytes III, spheroechinocytes and spherocytes (Figure 2E) while the CFDA-SE^{low} subpopulation was composed mostly of discocytes and echinocytes I and II (Figure 2F). Reciprocally, selection of SMEs and morphologically-normal RBCs (by their projected surface area) confirmed that most SMEs are CFDA-SE^{high} and that morphologically-normal RBCs are CFDA-SE^{low} RBCs (Figure S1). The gating strategy to segregate CFDA-SE^{low} and CFDA-SE^{high} subpopulations could be improved by using a size parameter (projected surface area), in addition of CFDA-SE intensity (Figure S2).

### CFDA-SE^{high} staining enables the quantification by flow cytometry of SMEs along storage

We next evaluated the evolution of RBC morphology during storage by flow cytometry using the CFDA-SE staining protocol and compared these observations to the proportion of SMEs detected by imaging flow cytometry on unstained cells (Roussel, 2017; Roussel, 2021). A CFDA-SE^{high} subpopulation (now gated using FSC-W and CFDA-SE intensity) accumulated along storage (Figure 3A) in the 8 RBC concentrates studied (grey line, Figure 3C). A SME subpopulation (using the nadir of the projected surface area bimodality) also accumulated along storage (Figure 3B) in the 8 RBC concentrates studied (black line, Figure 3C). For all donors, the CFDA-SE^{high} subpopulation accumulated upon storage from 3.3% ± 1.4% on day 3 to 47.2% ± 18.8% on day 42 (grey line, Figure 3C), with marked interdonor variability. Similarly, the SME subpopulation accumulated upon storage from 1.3 % ± 0.8% on day 3 to 38.7% ± 22.9% on day 42 (black line, Figure 3C). The proportion of morphologically-altered RBCs followed a similar evolution along storage with both techniques, increasing more rapidly after day 21. The proportion of CFDA-SE^{high} RBCs was slightly higher than the proportion of SMEs at each time point, reaching statistical significance on day 3 and 35 of storage. There was a very strong correlation between the proportion of CFDA-SE^{high} RBCs determined using flow cytometry and the proportion of SMEs determined by imaging flow cytometry (p < 0.0001; Spearman r = 0.93; r² = 0.88; Figure 3D).

### Enriched preparations of morphologically-normal and SMEs subpopulations of stored RBCs can be obtained by flow cytometry sorting

A flow cytometry gating strategy was next used to sort CFDA-SE^{low} and CFDA-SE^{high} RBC subpopulations (Figure 4A). Imaging flow cytometry was used to evaluate the content of each preparation, using fluorescence intensity and projected surface area. In Figure 4B a representative density plots of unsorted (middle panel), sorted CFDA-SE^{low} (left panel) and CFDA-SE^{high} (right panel) subpopulations illustrates the average purity obtained. In 5 separate experiments conducted on RBCs stored 42 days, preparations for CFDA-SE^{low} (left panel) and CFDA-SE^{high} (right panel) contained respectively a mean proportion of RBCs of interest of 96.4% ± 1.4% and 97.9% ± 2.0%. Scanning electron microscopy images confirmed that a majority of morphologically-normal RBCs (as discocytes and echinocytes I) were found in CFDA-SE^{low} subpopulations (Figure 4C, left panel) while a majority of SMEs (echinocytes III, spheroechinocytes and spherocytes) were present in CFDA-SE^{high} subpopulation (right panel) with a mix of these morphologies in the unsorted fraction (middle panel).

### REFERENCES:

Throughout this application, various references describe the state of the art to which this invention pertains. The disclosures of these references are hereby incorporated by reference into the present disclosure.

## Claims

1. A method for detecting the presence of storage-induced microerythrocytes (SMEs) in a red blood cell containing composition comprising the steps of i) staining the composition with an amount of a cell permeable dye, ii) incubating the stained composition for a sufficient period of time and under conditions suitable for allowing a bimodality of staining to appear in the red blood cells, and iii) distinguishing the cells by their intensity of staining wherein the subpopulation of SMEs are **characterized by** a high intensity of staining.

2. The method of claim 1 wherein the red blood cell containing composition is an individual stored red blood cell (RBC) unit.

3. The method of claim 1 wherein the dye is a fluorescent dye.

4. The method of claim 1 wherein the dye is an amine tracer.

5. The method of claim 1 wherein the dye is a succinimidyl ester-based dye that binds to the amine groups of cellular proteins.

6. The method of claim 6 the dye is selected from the group consisting of carboxyfluorescein diacetate succinimidyl ester (CFDA-SE), carboxyfluorescein succinimidyl ester (CFSE), carboxyeosin diacetate succinimidyl ester or any derivatives thereof.

7. The method of claim 6 wherein the dye is carboxyfluorescein diacetate succinimidyl ester (CFDA-SE).

8. The method of claim 1 wherein the dye is provided to the cells at a concentration ranging from about 0.01µM to about 1µM, preferably 0,05µM.

9. The method of claim 1 wherein the incubation step is carried out at 37°C and for at least 10, 15, 20, 25 or 30 minutes preferably 20 minutes.

10. The method of claim 3 wherein the fluorescence emitted by the fluorescent dye is measured by means of a flow cytometer.

11. The method of claim 1 that further comprises the step of assessing the size of the cells wherein the SMEs are **characterized by** a small size and a high intensity of staining.

12. The method of claim 1 that further comprises the step of quantifying the amount of SMEs present in the red blood cell containing composition.

13. A method of assessing the storage quality of a red blood cell containing solution comprising quantifying the amount of SMEs present in the composition by the quantifying method of claim 1 wherein the amount of SMEs correlates with the quality storage.

14. The method of claim 13 wherein the higher is the amount of SMEs present in the red blood cell composition, the worse is the storage quality of the red blood cell composition.

15. A method of predicting the transfusion recovery of a patient transfused with a red blood cell containing composition comprising quantifying the amount of SMEs present in the composition by the quantifying method of claim 1 wherein the amount of SMEs correlates with the recovery yield.

16. The method of claim 15 that comprises the steps of i) quantifying the amount of SMEs present in the red blood cell containing solution, ii) comparing the amount quantified at step i) with a predetermined reference value, iii) and concluding that the patient will a have a good recovery when the amount quantified at step i) is lower than the predetermined reference value or concluding that the patient will have a poor recovery when the amount quantified at step i) is higher than the predetermined reference value.

17. Use of the method of claim 1 for preparing a red blood cell containing composition suitable for transfusion comprising i) detecting the presence of SMEs in red blood cell containing composition and ii) separating the SMEs from blood cell containing composition.
